(19) Europäisches Patentamt
European Patent Office
Office européen des brevets

(11) Publication number: 0 030 009
B1

(12) EUROPEAN PATENT SPECIFICATION

(45) Date of publication of patent specification: 11.05.83

(21) Application number: 80107404.8

(22) Date of filing: 27.11.80

(51) Int. Cl.³: C 07 C 103/58, C 07 C 102/04, C 07 D 213/40, C 07 D 213/75, A 61 K 31/225, A 61 K 31/44

# (54) New derivatives of the 9-cis-6,6'-diapo-psi,psi-carotenedioic acid, preparation thereof and pharmaceutical compositions containing same.

(30) Priority: 30.11.79 IT 2774879

(43) Date of publication of application: 10.06.81 Bulletin 81/23

(45) Publication of the grant of the patent: 11.05.83 Bulletin 83/19

(84) Designated Contracting States: AT BE CH DE FR GB IT LI LU NL SE

(56) References cited:
GB - A - 1 254 771
US - A - 4 024 151

(73) Proprietor: Simes S.p.A.
Via Bellerio 41
I-20161 Milano (IT)

(72) Inventor: Ferrari, Giorgio
Via Borgonuovo, 7
I-20121 Milano (IT)
Inventor: Vecchietti, Vittorio
Via Spartaco, 23
I-20135 Milano (IT)

(74) Representative: Marchi, Massimo et al,
c/o Zambon S.p.A. Patent & Licensing Dept. Via Lillo del Duca, 10
I-20091 Bresso (Milano) (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 030 009 B1

New derivatives of the 9-cis-6,6′-diapo-psi,psi-carotenedioic acid, preparation thereof and pharmaceutical compositions containing same.

The present invention provides to new derivatives of the 9-cis-6,6′-diapo-ψψ-carotenedioic acid, their preparation and the pharmaceutical compositions containing them.

More particularly, the present invention provides new derivatives the 9-cis-6,6′-diapo-ψ-carotenedioic acid of the general formula (I)

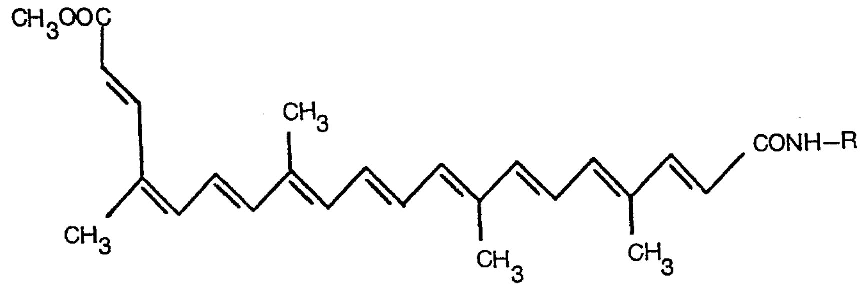

(I)

wherein R is a radical selected from the group comprising

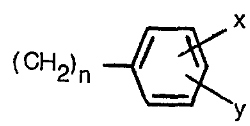

(wherein n = 0, 1, 2 and x, y, the same or different, are H, halogen, alkoxy, hydroxy, alkyl or trifluoromethyl radical);
2,3-pyridyl; 4-pyridyl;
2,3-pyridyl-methyl; 3-pyridyl-methyl.

More specifically, the present invention provides the following compounds comprised in the general formula (I)

1) 9-cis-6,6′-diappo-ψψ-carotenedioic acid, benzylamide monomethyl ester;
2) 9-cis-6,6′-diapo-ψψ-carotenedioic acid, 3,4-dimethoxy-benzylamide monomethyl ester;
3) 9-cis-6,6′-diapo-ψψ-carotenedioic acid, phenethylamide monomethylester;
4) 9-cis-6,6′-diapo-ψψ-carotenedioic acid, 3,4-dimethoxyphenethylamide monomethyl ester;
5) 9-cis-6,6′-diapo-ψψ-carotenedioic acid, anilide monomethylester;
6) 9-cis-6,6′-diapo-ψψ-carotenedioic acid, p-methoxyanilide monomethylester;
7) 9-cis-6,6′-diapo-ψψ-carotenedioic acid, p-chloroanilide monomethylester;
8) 9-cis-6,6′-diapo-ψψ-carotenedioic acid, 3,4-dichloroanilide monomethylester;
9) 9-cis-6,6′-diapo-ψψ-carotenedioic acid, 2,5-dimethoxyanilide monomethylester;
10) 9-cis-6,6′-diapo-ψψ-carotenedioic acid, 2,3-dimethylanilide monomethylester;
11) 9-cis-6,6′-diapo-ψψ-carotenedioic acid, p-hydroxyanilide monomethylester;
12) 9-cis-6,6′-diapo-ψψ-carotenedioic acid, m-hydroxyanilide monomethylester;
13) 9-cis-6,6′-diapo-ψψ-carotenedioic acid, o-hydroxyanilide monomethylester;
14) 9-cis-6,6′-diapo-ψψ-carotenedioic acid, m-fluoroanilide monomethylester;
15) 9-cis-6,6′-diapo-ψψ-carotenedioic acid, p-fluoroanilide monomethylester;
16) 9-cis-6,6′-diapo-ψψ-carotenedioic acid, o-fluoroanilide monomethylester;
17) 9-cis-6,6′-diapo-ψψ-carotenedioic acid, 4-pyridylamide monomethylester;
18) 9-cis-6,6′-diapo-ψψ-carotenedioic acid, 3-pyridylmethylamide monomethylester;
19) 9-cis-6,6′-diapo-ψψ-carotenedioic acid, 3-trifluoromethylanilide monomethylester;

The new products of this invention are endowed with antiproliferating activity.

As far as this activity is concerned, compound 18, tested "in vivo", has proved particularly useful.

By carrying out the test as indicated hereinbelow, the average survival time has been evaluated for mice (strain $CD_2F_1$) treated, or not, with compound 18.

Lymphocytic leukaemia was induced in the mice to be tested (i.p. inoculum; number of injected cells $10^6$; tissue: ascitic fluid).

Three groups each consisting of 6 female mice have been treated. The compound has been injected using as vehicle a physiological solution with TWEEN—80 at dosages of 200, 100 and 50 mg/kg respectively.

The drug has been injected once a day for 9 days from the day immediately after the inoculum. The used controls were 32.

The mortality of both treated mice and controls calculated on the treatment days is summarized in the following table:

| Days | No. of dead animals after treatment with | | | No. of dead controls |
|---|---|---|---|---|
| | 200 mg/kg | 100 mg/kg | 50 mg/kg | |
| VI | | | | |
| VII | | | | |
| VIII | 1 | | | 8 |
| IX | | | | 15 |
| X | | | | 5 |
| XI | | | 3 | 4 |
| XII | 3 | 4 | 3 | |
| XIII | 2 | 2 | | |
| XIV | | | | |

Therefore, the average survival times are as follows:

| Dosage | survival time (days) |
|---|---|
| 200 mg | 12.3 |
| 100 mg | 12.4 |
| 50 mg | 11.7 |
| control | 9.1 |

The percentage ratio (T/C) between the average survival time of the treated animals and that one of the controls is 135, 136, 128% respectively for dosages of 200, 100 and 50 mg/kg respectively. The new compounds of the present invention can be prepared by reacting the compound of formula (II)

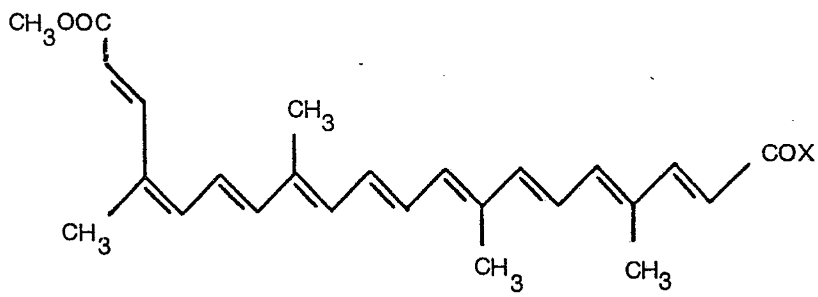

(II)

wherein X is —OH, Cl or the group O—CO—O—$R_1$, $R_1$ being $CH_3$, $C_2H_5$,$C_4H_7$ or $CH_2CCl_3$, is reacted with a compound R—$NH_2$ (wherein R has the previously specified meanings).

When X is —OH the reaction may be carried out in an aprotic polar solvent selected from $CHCl_3$, —$CH_2Cl_2$, tetrahydrofurane and dimethylsulfoxide, in the presence of an activator for the carboxylic group, such as for instance bicyclohexylcarbodiimide, at a temperature comprised between −10°C and +50°C.

When X is Cl, the acid chloride may be prepared by reacting the compound wherein X = OH with $PCl_3$ in a chlorinated solvent selected from $CHCl_3$, $CH_2$—$Cl_2$ and $Cl_2$—CH—CH—$Cl_2$ and then allowing the thus formed acid chloride to react in situ with R—$NH_2$ in excess.

When X is O—C—O—$R_1$ the acid may be reacted in the presence of an aprotic solvent selected from $CH_2Cl_2$, dioxane, tetrahydrofurane, diglyme, dimethylsulfoxide, dimethylformamide, first with an

organic base selected from triethylamine, diisopropylethylamine and dimethylaniline and thereafter with a derivative of the chlorocarbonic acid of the general formula Cl—CO—$OR_1$ (wherein $R_1$ has the above specified meaning), at temperatures comprised between −10°C and 0°C.

The thus obtained mixed anhydride may be then allowed to react, in the same solvent where it has been formed, with 2 molar eq. of the amine R—$NH_2$, at temperatures comprised between 10°C and 80°C to yield the desired compounds.

The new compounds of the present invention are crystalline substances endowed with well defined physico-chemical characteristics and colour generally varying from orange to dark red.

For the application in the pharmaceutical field, the compounds of the invention can be made up in a suitable form for topic and systemic use (oral and injectable administration, suppositories).

To this purpose the compounds of the invention can be formulated as compositions containing, besides the active substance, the generally used solvents, excipients, adjuvants, stabilizers and vehicles.

The dosages of active substance are comprised between 1 and 500 mg.

The following examples are given to illustrate the invention without limiting it in any way.

## Example 1

500 mg of monomethylester of the 9-cis-6,6′-diapo-ψψ-carotenedioic acid and 136 mg of benzylamine are suspended in 10 ml of $CH_2Cl_2$ and added with 265 mg of bicyclohexylcarbodiimide.

The thus obtained solution is left to stand for 15 h at room temperature and thereafter it is diluted with 40 ml $CH_2Cl_2$. The bicyclohexylcarbourea is filtered off and then the solution is washed with diluted HCl and water, dried on $Na_2SO_4$ and evaporated to dryness. The residue is analysed by chromatography on silica gel using as eluent $CH_2Cl_2$ containing 0.5% methanol.

By crystallization from acetone, 300 mg of 9-cis-6,6′-diapo-ψψ-carotenedioic acid, benzylamide monomethylester (compound 1) are obtained.

m.p. = 173—175°C; $M^+$ 469.

The analysis for $C_{31}H_{35}NO_3$ gives the following results:

| | C | H | N |
|---|---|---|---|
| calculated (%) | 79.28 | 7.51 | 2.98 |
| found (%) | 78.53 | 7.47 | 2.86 |

$\lambda_{max}^{MeOH}$ 494 mμ, 464 mμ

By working as above described, there are prepared the following compounds:

— compound 2

9-cis-6,6′-diapo-ψψ-carotenedioic acid, 3,4-dimethyoxy-benzylamide monomethylester.

m.p. = 193—195°C ($CHCl_3$/acetone) $M^+$ 543.

The analysis for $C_{34}H_{41}NO_5$ gives the following results:

| | C | H | N |
|---|---|---|---|
| calculated (%) | 75.11 | 7.60 | 2.58 |
| found (%) | 74.34 | 7.37 | 2.65 |

$\lambda_{max}^{MeOH}$ 488 mμ, 459 mμ

— compound 3

9-cis-6,6′-diapo-ψψ-carotenedioic acid, phenethylamide monomethylester.

m.p. = 192—194°C (dioxane) $M^+$ 497.

The analysis for $C_{33}H_{39}NO_3$ gives the following results:

| | C | H | N |
|---|---|---|---|
| calculated (%) | 79.64 | 7.90 | 7.81 |
| found (%) | 79.13 | 7.53 | 2.69 |

$\lambda_{max}^{MeOH}$ 488 mμ, 459 mμ

— compound 4

9-cis-6,6′-diapo-ψψ-carotenedioic acid, 3,4-dimethoxyphenethylamide monomethylester.

m.p. = 200—201°C ($CHCl_3$/acetone) $M^+$ 557.

The analysis for $C_{35}H_{43}NO_5$ gives the following results:

| | C | H | N |
|---|---|---|---|
| calculated (%) | 75.37 | 7.77 | 2.51 |
| found (%) | 75.00 | 7.42 | 2.42 |

$\lambda_{max}^{MeOH}$ 488 mμ, 459 mμ

Example 2

5 g of finely grounded 9-cis-6,6′-diapo-ψψ-carotenedioic acid, monomethylester are suspended in 250 ml of $CH_2Cl_2$ cooled to 0°C and treated with 1.17 g of $PCl_3$.

After 15 h at room temperature the mixture is again cooled to 0°C and 4.8 g of aniline are added. After 2 h at room temperature the reaction mixture is poured into 500 g of ice containing 30 ml of concentrated HCl.

The organic layer is separated, washed with water, with a $NaHCO_3$ solution and again with water to neutral reaction and finally is dried on $Na_2SO_4$ and concentrated to dryness.

The residue is analysed by chromatography on silica gel.

As eluent $CH_2Cl_2$ is first used and then $CH_2Cl_2$ containing 1% methanol. Using this last mixture unitary fractions are obtained which by evaporation and crystallization from acetone yield 1.6 g of 9-cis-6,6′-diapo-ψψ-carotenedioic acid, anilide monomethylester (compound 5).

m.p. 173—175°C; $M^+$ 469.
The analysis for $C_{31}H_{35}NO_3$ gives the following results:

| | C | H | N |
|---|---|---|---|
| calculated (%) | 79.28 | 7.51 | 2.98 |
| found (%) | 78.33 | 7.37 | 2.86 |

$\lambda_{max}^{MeOH}$ 494 mμ, 464 mμ

Example 3

5 g of 9-cis-6,6′-diapo-ψψ-carotenedioic acid, monomethylester are suspended in 50 ml dioxane and treated with 1.5 g triethylamine. The thus obtained solution is cooled to +5°C and added with 1.5 g of methylchloroformiate. The solution is stirred at room temperature for 1.5 h; thereafter 3.0 g of p-anisidine are added.

After 24 h at room temperature the reaction is completed.

The solution is evaporated to dryness and the residue is treated with acetone/ether; the thus formed solid is filtered off, washed with ether and crystallized from acetone.

2.5 g of 9-cis-6,6′-diapo-ψψ-carotenedioic acid, p-methoxyanilidine monomethylester (compound 6) are obtained.

m.p. = 177—179°C, $M^+$ 499.
The analysis for $C_{32}H_{37}NO_4$ gives the following results:

| | C | H | N |
|---|---|---|---|
| calculated (%) | 76.92 | 7.46 | 2.80 |
| found (%) | 75.63 | 7.38 | 2.66 |

$\lambda_{max}^{MeOH}$ 494 mμ, 464 mμ

By analogously working there are prepared the hereinbelow described compounds.

— compound 7
9-cis-6,6′-diapo-ψψ-carotenedioic acid, p-chloroanilide monomethylester.

m.p. = 178—180°C (acetone); $M^+$ 505, 503.
The analysis for $C_{31}H_{34}ClNO_3$ gives the following results:

| | C | H | N |
|---|---|---|---|
| calculated (%) | 78.87 | 6.80 | 2.78 |
| found (%) | 72.19 | 6.62 | 2.58 |

$\lambda_{max}^{MeOH}$ 494 mμ, 464 mμ

— compound 8
9-cis-6,6′-diapo-ψψ-carotenedioic acid, 3,4-dichloroanilide monomethylester.

m.p. = 170—172 °C (acetone); $M^+$ 539, 537.
The analysis for $C_{31}H_{33}ClNO_3$ gives the following results:

| | C | H | N |
|---|---|---|---|
| calculated (%) | 69.14 | 6.18 | 2.60 |
| found (%) | 68.56 | 5.97 | 2.45 |

$\lambda_{max}^{MeOH}$ 487 mμ, 459 mμ

— compound 9
9-cis-6,6'-diapo-ψψ-carotenedioic acid, 2,5-dimethoxyanilide monomethylester.

m.p. = 152—155°C (acetone); $M^+$ 529.
The analysis for $C_{33}H_{39}NO_5$ gives the following results:

| | C | H | N |
|---|---|---|---|
| calculated (%) | 74.83 | 7.42 | 2.64 |
| found (%) | 74.11 | 7.76 | 2.69 |

$\lambda_{max}^{MeOH}$ 494 mμ, 464 mμ

— compound 10
9-cis-6,6'-diapo-ψψ-carotenedioic acid, 2,3-dimethylanilide monomethylester.

m.p. = 187—188°C (acetone), $M^+$ 497
The analysis for $C_{33}H_{39}NO_3$ gives the following results:

| | C | H | N |
|---|---|---|---|
| calculated (%) | 76.94 | 7.90 | 2.81 |
| found (%) | 78.56 | 7.71 | 2.63 |

$\lambda_{max}^{MeOH}$ 488 mμ, 460 mμ

— compound 11
9-cis-6,6'-diapo-ψψ-carotenedioic acid, p-hydroxyanilide monomethylester.

m.p. = 210—211°C (dioxane); $M^+$ 485
The analysis for $C_{31}H_{35}NO_4$ gives the following results:

| | C | H | N |
|---|---|---|---|
| calculated (%) | 76.67 | 7.26 | 2.88 |
| found (%) | 75.05 | 7.15 | 2.65 |

$\lambda_{max}^{MeOH}$ 494 mμ, 464 mμ

— compound 12
9-cis-6,6'-diapo-ψψ-carotenedioic acid, m-hydroxyanilide monomethylester.

m.p. = 149—152°C (acetone); $M^+$ 485
The analysis for $C_{31}H_{35}NO_4$ gives the following results:

| | C | H | N |
|---|---|---|---|
| calculated (%) | 76.67 | 7.26 | 2.88 |
| found (%) | 75.97 | 7.43 | 2.80 |

$\lambda_{max}^{MeOH}$ 490 mμ, 462 mμ

— compound 13
9-cis-6,6'-diapo-ψψ-carotenedioic acid, o-hydroxyanilide monomethylester.

m.p. = 208—210°C (acetone); $M^+$ 485
The analysis for $C_{31}H_{35}NO_4$ gives the following results:

| | C | H | N |
|---|---|---|---|
| calculated (%) | 76.67 | 7.26 | 2.88 |
| found (%) | 75.68 | 7.66 | 2.86 |

$\lambda_{max}^{MeOH}$ 492 mμ, 464 mμ

— compound 14
9-cis-6,6′-diapo-ψψ-carotenedioic acid, m-fluoroanilide monomethylester.

m.p. = 171—172°C (acetone); $M^+$ 487
The analysis for $C_{31}H_{34}FNO_3$ gives the following results:

| | C | H | N |
|---|---|---|---|
| calculated (%) | 76.36 | 7.03 | 2.87 |
| found (%) | 75.52 | 6.98 | 2.79 |

$\lambda_{max}^{MeOH}$ 494 mμ, 462 mμ

— compound 15
9-cis-6,6′-diapo-ψψ-carotenedioic acid, p-fluoroanilide monomethylester.

m.p. = 170—171°C (acetone); $M^+$ 187
The analysis for $C_{31}H_{34}FNO_3$ gives the following results:

| | C | H | N |
|---|---|---|---|
| calculated (%) | 76.36 | 7.03 | 2.87 |
| found (%) | 76.25 | 6.91 | 2.56 |

$\lambda_{max}^{MeOH}$ 490 mμ; 462 mμ

— compound 16
9-cis-6,6′-diapo-ψψ-carotenedioic acid, o-fluoroanilide monomethylester.

m.p. = 182—183°C (acetone); $M^+$ 187
The analysis for $C_{31}H_{34}FNO_3$ gives the following results:

| | C | H | N |
|---|---|---|---|
| calculated (%) | 76.36 | 7.03 | 2.87 |
| found (%) | 76.13 | 6.90 | 2.98 |

$\lambda_{max}^{MeOH}$ 494 mμ, 462 mμ

— compound 17
9-cis-6,6′-diapo-ψψ-carotenedioic acid, 4-pyridylamide monomethylester.

m.p. = 192—194°C (acetone); $M^+$ 470
The analysis for $C_{30}H_{34}N_2O_3$ gives the following results:

| | C | H | N |
|---|---|---|---|
| calculated (%) | 76.56 | 7.28 | 5.95 |
| found (%) | 75.04 | 7.33 | 5.57 |

$\lambda_{max}^{MeOH}$ 490 mμ, 468 mμ

— compound 18
9-cis-6,6′-diapo-ψψ-carotenedioic acid, 3-pyridylmethylamide monomethylester.

m.p. = 197—199°C ($CHCl_3$/acetone); $M^+$ 484
The analysis for $C_{31}H_{36}N_2O_3$ gives the following results:

| | C | H | N |
|---|---|---|---|
| calculated (%) | 76.83 | 7.49 | 5.78 |
| found (%) | 76.84 | 7.26 | 5.78 |

$\lambda_{max}^{MeOH}$ 489 mμ, 460 mμ

— compound 19
9-cis-6,6′-diapo-ψψ-carotenedioic acid, 3-trifluoromethylanilide monomethylester.

m.p. = 172—175°C (acetone); $M^+$ 537
The analysis for $C_{32}H_{34}F_3O_3$ gives the following results:

| | C | H | N |
|---|---|---|---|
| calculated (%) | 71.49 | 6.37 | 2.61 |
| found (%) | 70.80 | 6.21 | 2.45 |

$\lambda_{max}^{MeOH}$ 494 mμ, 462 mμ

**Claims**

1. Derivatives of the 9-cis-6,6'-diapo-ψψ-carotenedioic acid of the general formula (I)

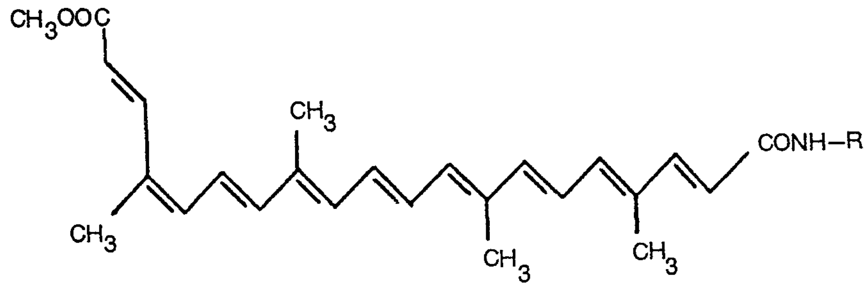

(I)

wherein R is a radical selected from the group comprising

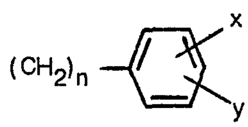

(wherein n = 0, 1, 2 and x, y, equal of different, are H, halogen, alkoxy, —OH, alkyl or trifluoromethyl radical);
2,3-pyridyl; 4-pyridyl;
2,3-pyridyl-methyl; 3-pyridyl-methyl.

2. 9-cis-6,6'-Diapo-ψψ-carotenedioic acid, benzylamide monomethylester.
3. 9-cis-6,6'-Diapo-ψψ-carotenedioic acid, 3,4-dimethoxy-benzylamide monomethylester.
4. 9-cis-6,6'-Diapo-ψψ-carotenedioic acid, phenethylamide monomethylester.
5. 9-cis-6,6'-Diapo-ψψ-carotenedioic acid, 3,4-dimethoxyphenethylamide monomethylester.
6. 9-cis-6,6'-Diapo-ψψ-carotenedioic acid, anilide monomethylester.
7. 9-cis-6,6'-Diapo-ψψ-carotenedioic acid, p-methoxyanilide monomethylester.
8. 9-cis-6,6'-Diapo-ψψ-carotenedioic acid, p-chloranilide monomethylester.
9. 9-cis-6,6'-Diapo-ψψ-carotenedioic acid, 3,4-dichloroanilide monomethylester.
10. 9-cis-6,6'-Diapo-ψψ-carotenedioic acid, 2,5-dimethoxyanilide monomethylester.
11. 9-cis-6,6'-Diapo-ψψ-carotenedioic acid, 2,3-dimethylanilide monomethylester.
12. 9-cis-6,6'-Diapo-ψψ-carotenedioic acid, p-hydroxyanilide monomethylester.
13. 9-cis-6,6'-Diapo-ψψ-carotenedioic acid, m-hydroxyanilide monomethylester.
14. 9-cis-6,6'-Diapo-ψψ-carotenedioic acid, o-hydroxyanilide monomethylester.
15. 9-cis-6,6'-Diapo-ψψ-carotenedioic acid, m-fluoroanilide monomethylester.
16. 9-cis-6,6'-Diapo-ψψ-carotenedioic acid, p-fluoroanilide monomethylester.
17. 9-cis-6,6'-Diapo-ψψ-carotenedioic acid, o-fluoroanilide monomethylester.
18. 9-cis-6,6'-Diapo-ψψ-carotenedioic acid, 4-pyridylmethylamide monomethylester.
19. 9-cis-6,6'-Diapo-ψψ-carotenedioic acid, 3-pyridylmethylamide monomethylester.
20. 9-cis-6,6'-Diapo-ψψ-carotenedioic acid, 3-trifluoromethylanilide monomethylester.
21. A process for the preparation of derivatives of the 9-cis-6,6'-diapo-ψψ-carotenedioic acid of the general formula (I)

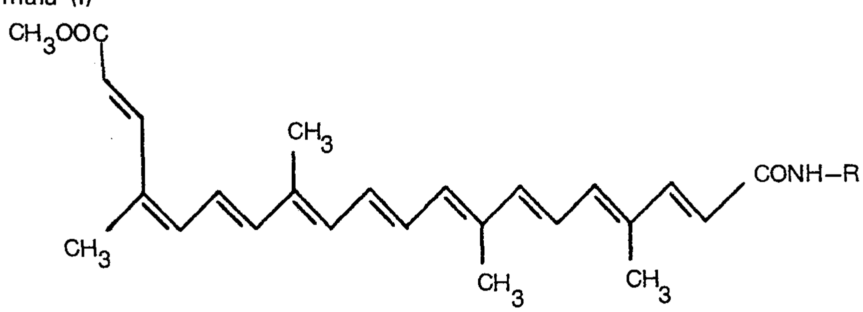

(I)

characterized in that a compound of the formula (II)

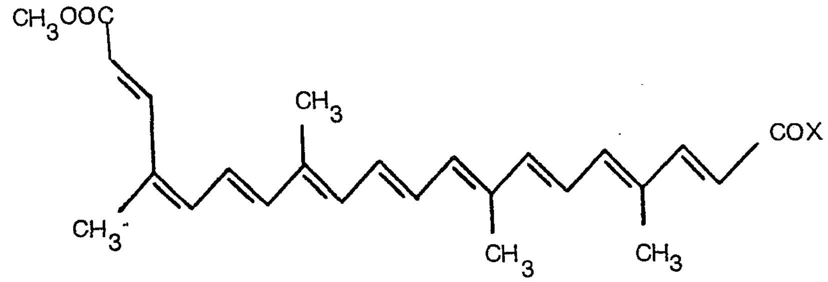

(II)

wherein X is —OH, Cl or the group O—CO—O—$R_1$, $R_1$ being $CH_3$, $C_2H_5$,$C_4H_7$ or $CH_2CCl_3$, is reacted with a compound R—$NH_2$ (wherein R has the above specified meanings).

22. A process according to claim 21, characterized in that when X = OH, the reaction is carried out in an aprotic solvent, in the presence of an activator for the carboxylic group, at a temperature comprised between −10°C and +50°C.

23. A process according to claim 22, characterized in that the aprotic solvent is selected from the class consisting of $CHCl_3$, $CH_2Cl_2$, tetrahydrofurane, dimethylsulfoxide.

24. A process according to claim 22, characterized in that the activator for the carboxylic group is bicyclohexylcarbodiimide.

25. A process according to claim 21, characterized in that when X = Cl, the acid chloride is prepared by reacting the compound (II) wherein X = OH with $PCl_3$, in chlorinated solvent, and thereafter the obtained acid chloride in situ with R—$NH_2$.

26. A process according to claim 25, characterized in that the chlorinated solvent is selected from the class consisting of $CHCl_3$, $CH_2Cl_2$, $Cl_2$—CH—CH—$Cl_2$.

27. A process according to claim 25, characterized in that the preparation of the acid chloride is carried out in the presence of R—$NH_2$ in excess.

28. A process according to claim 21, characterized in that when X is O—CO—O—$R_1$, the compound (II) wherein X = OH is reacted in the presence of an aprotic solvent, first with an organic base and thereafter, at a temperature comprised between −10°C and 0°C, with a derivative of the chlorocarbonic acid of the formula

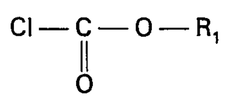

wherein $R_1$ has the previously specified meaning, the mixed anhydride being the allowed to react with R—$NH_2$ (wherein R has the meaning specified in claim 1) at a temperature comprised between 10° and 80°C.

29. A process according to claim 28, characterized in that the aprotic solvent is selected from $CH_2Cl_2$, dioxane, tetrahydrofurane, diglyme, dimethylsulfoxide and dimethylformamide and the organic base is selected from diisopropylethylamine and dimethylaniline.

30. A process according to claim 28, characterized in that the reaction between the mixed anhydride and R—$NH_2$ occurs in the same solvent wherein the mixed anhydride has been formed and with 2 molar eq. of the amine.

31. Pharmaceutical compositions containing as active ingredient a compound of the general formula (I) together with pharmaceutically acceptable excipients and vehicles.

**Patentansprüche**

1. 9-cis-6,6′-Diapo-ψψ-carotindicarbonsäurederivative der allgemeinen Formel (I)

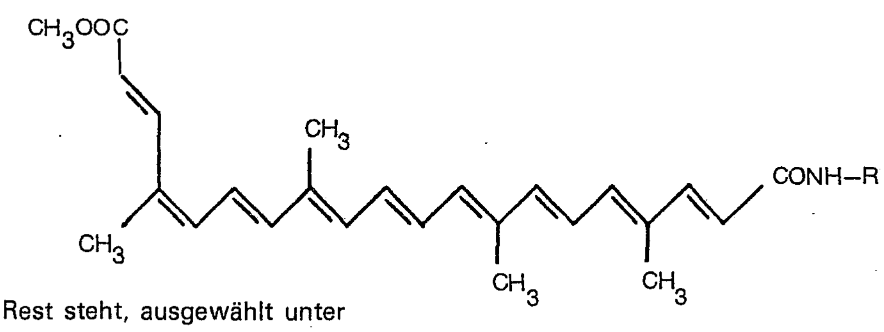

(I)

worin

R für einen Rest steht, ausgewählt unter

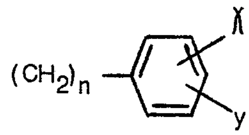

(worin n = 0, 1, 2 und x, y, die gleich oder verschieden sind, für H, Halogen, einen Alkoxy-, OH, Alkyl- oder Trifluoromethylrest stehen);
2,3-Pyridyl; 4-Pyridyl;
2,3-Pyridyl-methyl; 3-Pyridyl-methyl.

2. 9-cis-6,6'-Diapo-ψψ-carotindicarbonsäure-benzylamid, monomethylester.

3. 9-cis-6,6'-Diapo-ψψ-carotindicarbonsäure-3,4-di-methoxybenzylamid, monomethylester.

4. 9-cis-6,6'-Diapo-ψψ-carotindicarbonsäurephenethylamid, monomethylester.

5. 9-cis-6,6'-Diapo-ψψ-carotindicarbonsäure-3,4-dimethoxyphenethylamid, monomethylester.

6. 9-cis-6,6'-Diapo-ψψ-carotindicarbonsäureanilid, monomethylester.

7. 9-cis-6,6'-Diapo-ψψ-carotindicarbonsäure-p-methoxyanilid, monomethylester.

8. 9-cis-6,6'-Diapo-ψψ-carotindicarbonsäure-p-chloranilid, monomethylester.

9. 9-cis-6,6'-Diapo-ψψ-carotindicarbonsäure-3,4-dichloranilid, monomethylester.

10. 9-cis-6,6'-Diapo-ψψ-carotindicarbonsäure-2,5-dimethoxyanilid, monomethylester.

11. 9-cis-6,6'-Diapo-ψψ-carotindicarbonsäure-2,3-dimethylanilid, monomethylester.

12. 9-cis-6,6'-Diapo-ψψ-carotindicarbonsäure-p-hydroxyanilid, monomethylester.

13. 9-cis-6,6'-Diapo-ψψ-carotindicarbonsäure-m-hydroxyanilid, monomethylester.

14. 9-cis-6,6'-Diapo-ψψ-carotindicarbonsäure-o-hydroxyanilid, monomethylester.

15. 9-cis-6,6'-Diapo-ψψ-carotindicarbonsäure-m-fluoranilid, monomethylester.

16. 9-cis-6,6'-Diapo-ψψ-carotindicarbonsäure-p-fluoranilid, monomethylester.

17. 9-cis-6,6'-Diapo-ψψ-carotindicarbonsäure-o-fluoranilid, monomethylester.

18. 9-cis-6,6'-Diapo-ψψ-carotindicarbonsäure-4-pyridylmethylamid, monomethylester.

19. 9-cis-6,6'-Diapo-ψψ-carotindicarbonsäure-3-pyridylmethylamid, monomethylester.

20. 9-cis-6,6'-Diapo-ψψ-carotindicarbonsäure-3-trifluormethylanilid, monomethylester.

21. Verfahren zur Herstellung der 9-cis-6,6'-Diapo-ψψ-carotindicarbonsäurederivate der allgemeinen Formel (I)

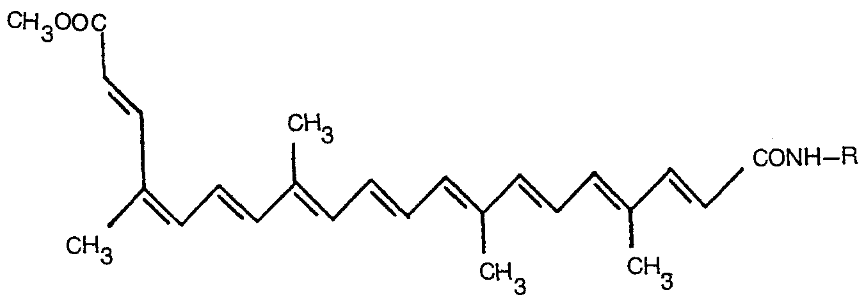

(I)

dadurch gekennzeichnet, daß man eine Verbindung der Formel (II)

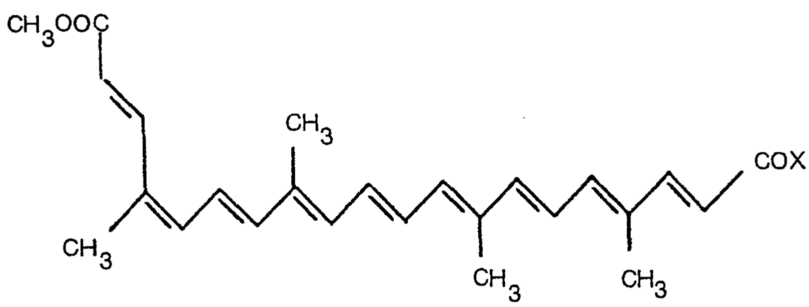

(II)

worin
X für —OH, Cl oder die Gruppe O—CO—O—$R_1$ steht, wobei $R_1$ $CH_3$, $C_2H_5$, $C_4H_7$ oder $CH_2CCl_3$ bedeutet, mit einer Verbindung R—$NH_2$ (worin R die oben angegebenen Bedeutungen besitzt) umsetzt.

22. Verfahren nach Anspruch 21, dadurch gekennzeichnet, daß man, wenn X = OH, die Reaktion in einem aprotischen Lösungsmittel, in Gegenwart eines Aktivierungsmittels für die Carboxylgruppe und bei einer Temperatur zwischen −10°C und +50°C durchführt.

23. Verfahren nach Anspruch 22, dadurch gekennzeichnet, daß das aprotische Lösungsmittel ausgewählt ist unter $CHCl_3$, $CH_2Cl_2$, Tetrahydrofuran, Dimethylsulfoxid.

24. Verfahren nach Anspruch 22, dadurch gekennzeichnet, daß das Aktivierungsmittel für die Carboxylgruppe Bicyclohexylcarbodiimid ist.

25. Verfahren nach Anspruch 21, dadurch gekennzeichnet, daß man, wenn X = Cl, das Säurechlorid durch Umsetzung der Verbindung (II), worin X = OH, mit $PCl_3$ in einem chlorierten Lösungsmittel herstellt und anschließend das erhaltene Säurechlorid in situ mit R—$NH_2$ umsetzt.

26. Verfahren nach Anspruch 25, dadurch gekennzeichnet, daß das chlorierte Lösungsmittel ausgewählt ist unter $CHCl_3$, $CH_2Cl_2$, $Cl_2$—CH—CH—$Cl_2$.

27. Verfahren nach Anspruch 25, dadurch gekennzeichnet, daß die Herstellung des Säurechlorids in Gegenwart von überschüssigem R—$NH_2$ erfolgt.

28. Verfahren nach Anspruch 21, dadurch gekennzeichnet, daß man, wenn X für O—CO—O—$R_1$ steht, die Verbindung (II), worin X = OH, in Gegenwart eines aprotischen Lösungsmittels zuerst mit einer organischen Base und anschließend bei einer Temperatur zwischen −10°C und 0°C mit einem Chlorcarbonsäurederivat der Formel

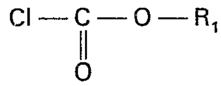

worin $R_1$ die oben angegebenen Bedeutungen besitzt, umsetzt und das gemischte Anhydrid dann mit R—$NH_2$ (worin R die in Anspruch 1 angegebenen Bedeutungen besitzt) bei einer Temperatur zwischen 10°C und 80°C reagieren läßt.

29. Verfahren nach Anspruch 28, dadurch gekennzeichnet, daß das aprotische Lösungsmittel ausgewählt ist unter $CH_2Cl_2$, Dioxan, Tetrahydrofuran, Diglyme, Dimethylsulfoxid und Dimethylformamid und die organische Base ausgewählt ist unter Diisopropylethylamin und Dimethylanilin.

30. Verfahren nach Anspruch 28, dadurch gekennzeichnet, daß die Reaktion zwischen dem gemischten Anhydrid und R—$NH_2$ im gleichen Lösungsmittel wie zur Bildung des gemischten Anhydrids und mit 2 Mol-Äquivalenten Amin erfolgt.

31. Pharmazeutische Mittel, enthaltend eine Verbindung der allgemeinen Formel (I) als Wirkstoff, zusammen mit pharmazeutisch verträglichen Excipientien und Trägern.

**Revendications**

1. Dérivés de l'acide 9-cis-6,6'-diapo-ψψ-carotène-dioïque de formule générale (I)

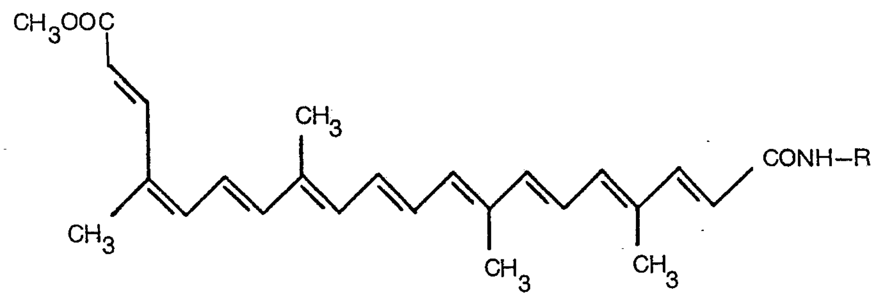

(I)

dans laquelle R est un radical choisi dans le groupe constitué par:

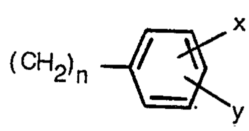

(avec n = 0, 1, 2 et x, y, semblables ou différents, représentant H, un halogène, un radical alcoxy, —OH, alcoyle ou trifluorométhyle),
2,3-pyridyle; 4-pyridyle;
2,3-pyridyl-méthyle; 3-pyridyl-méthyle.

2. Ester monométhylique de benzylamide d'acide 9-cis-6,6'-diapo-ψψ-carotène-dioïque.

3. Ester monométhylique de 3,4-diméthoxy-benzylamide d'acide 9-cis-6,6'-diapo-ψψ-carotène-dioïque.

4. Ester monométhylique de phénétylamide d'acide 9-cis-6,6'-diapo-ψψ-carotène-dioïque.

5. Ester monométhylique de 3,4-diméthoxyphénéthylamide d'acide 9-cis-6,6'-diapo-ψψ-carotène-dioïque.

6. Ester monométhylique d'anilide d'acide 9-cis-6,6'-diapo-ψψ-carotène-dioïque.

7. Ester monométhylique de p-méthoxyanilide d'acide 9-cis-6,6'-diapo-ψψ-carotène-dioïque.

8. Ester monométhylique de p-chloranilide d'acide 9-cis-6,6'-diapo-ψψ-carotène-dioïque.

9. Ester monométhylique de 3,4-dichloranilide d'acide 9-cis-6,6'-diapo-ψψ-carotène-dioïque.

10. Ester monométhylique de 2,5-diméthoxyanilide d'acide 9-cis-6,6'-diapo-ψψ-carotène-dioïque.

11. Ester monométhylique de 2,3-diméthylanilide d'acide 9-cis-6,6'-diapo-ψψ-carotène-dioïque.

12. Ester monométhylique de p-hydroxyanilide d'acide 9-cis-6,6'-diapo-ψψ-carotène-dioïque.

13. Ester monométhylique de m-hydroxyanilide d'acide 9-cis-6,6'-diapo-ψψ-carotène-dioïque.

14. Ester monométhylique d'o-hydroxyanilide d'acide 9-cis-6,6'-diapo-ψψ-carotène-dioïque.

15. Ester monométhylique de m-fluoranilide d'acide 9-cis-6,6'-diapo-ψψ-carotène-dioïque.

16. Ester monométhylique de p-fluoranilide d'acide 9-cis-6,6'-diapo-ψψ-carotène-dioïque.

17. Ester monométhylique d'o-fluoranilide d'acide 9-cis-6,6'-diapo-ψψ-carotène-dioïque.

18. Ester monométhylique de 4-pyridylméthylamide d'acide 9-cis-6,6'-diapo-ψψ-carotène-dioïque.

19. Ester monométhylique de 3-pyridylméthylamide d'acide 9-cis-6,6'-diapo-ψψ-carotène-dioïque.

20. Ester monométhylique de 3-trifluorométhylanilide d'acide 9-cis-6,6'-diapo-ψψ-carotène-dioïque.

21. Procédé pour la préparation de dérivés de l'acide 9-cis-6,6'-diapo-ψψ-carotène-dioïque de formule générale (I)

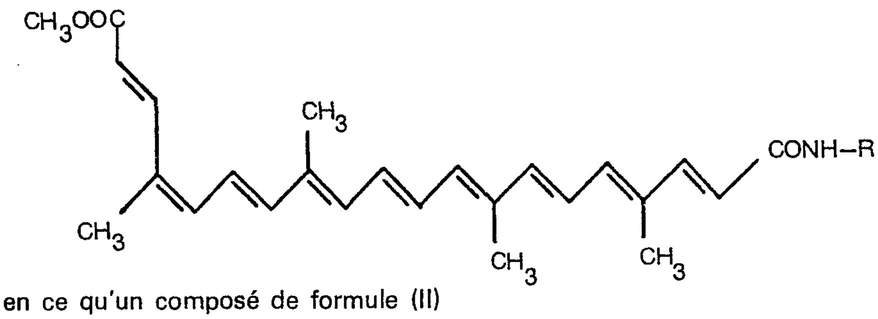

(I)

caractérisé en ce qu'un composé de formule (II)

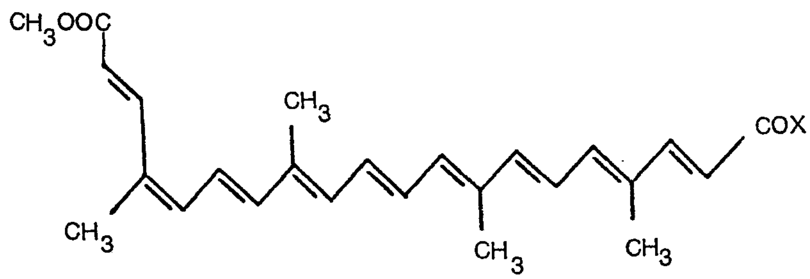

(II)

dans laquelle X représente —OH, Cl ou le groupe O—CO—O—$R_1$ ($R_1$ étant $CH_3$, $C_2H_5$, $C_4H_7$ ou $CH_2CCl_3$) est mis à réagir avec un composé de formule R—$NH_2$ (dans laquelle R a la signification donnée ci-dessus).

22. Procédé selon la revendication 21, caractérisé en ce qu'au cas où X = OH, la réaction est menée dans un solvant aprotique, en présence d'un activant pour le groupe carboxylique, à une température comprise entre −10°C et +50°C.

23. Procédé selon la revendication 22, caractérisé en ce que le solvant aprotique est choisi dans la catégorie constituée par $CHCl_3$, $CH_2Cl_2$, le tétrahydrofuranne, le diméthylsulfoxyde.

24. Procédé selon la revendication 22, caractérisé en ce que l'activant pour le groupe carboxylique est le bicyclohexylcarbodiimide.

25. Procédé selon la revendication 21, caractérisé en ce qu'au cas où X = Cl, le chlorure d'acide est préparé par la mise en réaction du composé (II) dans lequel X = OH avec $PCl_3$, dans un solvant chloré, puis la mise en réaction du chlorure d'acide obtenu avec R—$NH_2$ in situ.

26. Procédé selon la revendication 25, caractérisé en ce que le solvant chloré est choisi dans la catégoris constituée par $CHCl_3$, $CH_2Cl_2$, $Cl_2$—CH—CH—$Cl_2$.

27. Procédé selon la revendication 25, caractérisé en ce que la préparation du chlorure d'acide est effectuée en présence de R—$NH_2$ en excès.

28. Procédé selon la revendication 21, caractérisé en ce qu'au cas où X = O—CO—O—$R_1$, le composé (II) dans lequel X = OH est mis en réaction, en présence d'un solvant aprotique, tout d'abord avec une base organique, puis, à une température comprise entre −10°C et 0°C, avec un dérivé de l'acide chlorocarbonique de formule

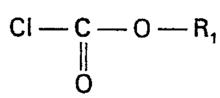

dans laquelle $R_1$ a la signification donnée ci-dessus, puis on laisse réagir l'anhydride mixte avec R—$NH_2$

(dans lequel R a la signification donnée dans la revendication 1) à une température comprise entre 10° et 80°C.

29. Procédé selon la revendication 28, caractérisé en ce que le solvant aprotique est choisi parmi $CH_2Cl_2$, le dioxanne, le tétrahydrofuranne, le diglyme, le diméthylsulfoxyde et le diméthylformamide et la base organique est choisie entre la diisopropyléthylamine et la diméthylaniline.

30. Procédé selon la revendication 28, caractérisé en ce que la réaction entre l'anhydride mixte et $R—NH_2$ s'effectue dans le même solvant dans lequel l'anhydride mixte a été formé et avec 2 équivalents molaires de l'amine.

31. Composition pharmaceutique, contenant en tant qu'élément actif un composé de formule générale (I), avec des excipients et véhicules acceptables pharmaceutiquement.